Europäisches Patentamt

European Patent Office

Office européen des brevets

(19)

(11) Veröffentlichungsnummer : **0 410 236 B1**

(12) **EUROPÄISCHE PATENTSCHRIFT**

(51) Int. Cl.⁵ : **A61K 31/015, A61K 9/14,
A61K 47/44, A23L 1/275**

(45) Veröffentlichungstag der Patentschrift :
**27.10.93 Patentblatt 93/43**

(21) Anmeldenummer : **90113500.4**

(22) Anmeldetag : **14.07.90**

(54) **Verfahren zur Herstellung von Carotinoidpräparaten.**

Die Akte enthält technische Angaben, die nach
dem Eingang der Anmeldung eingereicht
wurden und die nicht in dieser Patentschrift
enthalten sind.

(30) Priorität : **25.07.89 CH 2778/89**

(43) Veröffentlichungstag der Anmeldung :
**30.01.91 Patentblatt 91/05**

(45) Bekanntmachung des Hinweises auf die
Patenterteilung :
**27.10.93 Patentblatt 93/43**

(84) Benannte Vertragsstaaten :
**AT BE CH DE DK FR GB IT LI NL**

(56) Entgegenhaltungen :
**EP-A- 0 239 086
US-A- 2 861 891
US-A- 4 844 934**

(73) Patentinhaber : **F. HOFFMANN-LA ROCHE AG
Postfach 3255
CH-4002 Basel (CH)**

(72) Erfinder : **Cathrein, Ernst
Hauptstrasse 95
CH-4147 Aesch (CH)**
Erfinder : **Stein, Hermann
Brüelmatten 13
CH-4410 Liestal (CH)**
Erfinder : **Stoller, Hansjörg
Niederbergstrasse 21
CH-4153 Reinach (CH)**
Erfinder : **Viardot, Klaus
Auf der Bischoffhöhe 36
CH-4125 Riehen (CH)**

(74) Vertreter : **Cottong, Norbert A. et al
Grenzacherstrasse 124 Postfach 3255
CH-4002 Basel (CH)**

**Beschreibung**

Die vorliegende Erfindung betrifft ein neues Verfahren zur Herstellung von kolloid-dispersen Carotinoidpräparaten Die erfindungsgemäss hergestellten Präparate dienen, je nach verwendetem Carotinoid, sowohl zur Herstellung von pharmazeutischen Anwendungsformen, als auch zum Färben von Lebensmitteln und als Futtermittelzusätze.

Es gibt Hinweise, dass z.B. β-Carotin als Prophylactikum gegen Krebserkrankungen wirksam ist. β-Carotin, und auch weitere Carotinoide wie z.B. Lycopen, Bixin, Zeaxanthin, Cryptoxanthin, Lutein, Canthaxanthin, Astaxanthin, β-apo-8'-Carotinal, β-apo-12'-Carotinal, sowie Ester von hydroxy- und carboxyhaltigen Verbindungen dieser Gruppe, z.B. die niederen Alkylester, vorzugsweise die Methyl- und Aethylester, haben zudem eine beträchtliche Bedeutung als Farbstoffe oder farbgebende Mittel für Lebensmittel oder auch als Futtermittelzusätze erlangt.

Carotinoide sind jedoch Substanzen, die in Wasser unlöslich sind, hohe Schmelzpunkte besitzen und darüber hinaus auch hitze- und oxydationsempfindlich sind.

Im Fall von z.B. β-Carotin bedingen diese Eigenschaften, insbesondere die Wasserunlöslichkeit, eine äusserst schlechte Bioverfügbarkeit aus dieses Carotinoid enthaltenden pharmazeutischen Darreichungsformen, wie etwa Tabletten und Kapseln. Die erwähnten Eigenschaften stehen zudem einer direkten Verwendung der kristallinen Materialien zum Färben von wässrigen Lebensmitteln, als Futterzusätze oder auch zur Verwendung als Quelle für Vitamin A entgegen, da die Materialien in dieser Form nur schlecht absorbiert werden oder nur schlechte Färbungseffekte ergeben. Die oben genannten Eigenschaften von Carotinoiden sind insbesondere beim Färben von wässrigen Medien nachteilig, da es als Folge ihrer Wasserunlöslichkeit äusserst schwierig ist, eine homogene oder ausreichend intensive Farbwirkung zu erreichen.

Aus der Literatur sind bereits verschiedenartige Verfahren zur Herstellung derartiger Präparate bekannt, welche jedoch mit jeweils mehr oder weniger grossen Nachteilen verbunden sind. So ist beispielsweise aus der deutschen Patentschrift No. 1 211 911 bekannt, Carotinoidpräparate dadurch herzustellen, dass man ein Carotinoid in einem Carotinoidlösungsmittel löst, die Lösung in einer wässrigen Lösung eines Schutzkolloides emulgiert und anschliessend aus dieser Emulsion das Lösungsmittel wieder entfernt. Der Nachteil dieses Verfahrens besteht darin, dass als Lösungsmittel vorzugsweise chlorierte Kohlenwasserstoffe verwendet werden und deren Entfernung letztlich technisch aufwendig ist. Weiterhin ist beispielsweise aus der europäischen Patentschrift No. 65 193 bekannt, Carotinoidpräparate dadurch herzustellen, dass man ein Carotinoid in einem nichtchlorierten flüchtigen, mit Wasser mischbaren organischen Lösungsmittel bei Temperaturen zwischen 50° und 200° C innerhalb einer Zeit von weniger als 10 Sekunden löst, aus der erhaltenen Lösung durch Mischen mit einer Lösung eines Kolloides das Carotinoid in kolloid-disperser Form ausfällt und anschliessend wiederum das Lösungsmittel entfernt. Auch hier muss also wieder ein organisches Lösungsmittel entfernt werden, was wiederum technisch aufwendig ist.

Ferner ist aus der US-Patentschrift Nr. 2 861 891 ein Verfahren zur Herstellung von wasserdispergierbaren Carotinoidpräparaten bekannt. Gemäss diesem Verfahren wird das Carotinoid in einem erhitzten, essbaren Oel bei Uebersättigung gelöst, die resultierende, noch warme, übersättigte Lösung in einer wässrigen gellierbaren Kolloidlösung, z.B. einer Gelatinlösung, emulgiert und schliesslich die so entstandene Emulsion in trockene Partikel, z.B. mittels Sprühtrocknung, übergeführt. Die europäische Patentpublikation Nr. 239 086 beschreibt ebenfalls die Herstellung von wasserdispergierbaren Carotinoidpräparaten, und zwar in diesem Fall durch Auflösen des Carotinoids in einem Trägeröl bei erhöhter Temperatur bis zur Sättigung, schnelles Emulgieren der resultierenden Oellösung in einem wässrigen Schutzkolloid und Entfernen des Wassers von der Emulsion, z.B. mittels Sprühtrocknung, wobei man als Schutzkolloid eine Mischung aus dem Ester einer langkettigen Fettsäure mit Ascorbinsäure und einem kaltwasserlöslichen Stärkeprodukt verwendet.

Es bestand somit ein Bedürfnis nach einem Verfahren zur Herstellung von Carotinoidpräparaten, welches ohne die Verwendung von organischen Lösungsmitteln auskommt, und dennoch Präparate liefert, welche leicht in wässrigen Medien dispergierbar sind und sich zudem - im Falle von β-Carotin - zur Herstellung von pharmazeutischen Darreichungsformen mit guter Bioverfügbarkeit des Wirkstoffes eignen.

Durch das erfindungsgemässe Verfahren ist es nunmehr gelungen, die geschilderten Nachteile zu vermeiden und Carotinoidpräparate mit den gewünschten Eigenschaften zu erhalten.

Das erfindungsgemässe Verfahren zur Herstellung von kolloid-dispersen Carotinoidpräparaten ist dadurch gekennzeichnet, dass man eine Suspension eines Carotinoides in einem hochsiedenden Oel während einer Zeitdauer von höchstens 30 Sekunden mit überhitztem Wasserdampf in Kontakt bringt, dass man das erhaltene Gemisch in eine wässrige Lösung eines Kolloides emulgiert und diese Emulsion anschliessend versprüht und trocknet.

Der Ausdruck "Carotinoid" umfasst im Rahmen der vorliegenden Erfindung alle bekannten und üblicherweise für die Lebensmittelfärbung und als Futtermittelzusätze in Frage kommenden Vertreter dieser Substanzklasse, insbesondere die eingangs erwähnten Verbindungen. Die in Frage kommenden Carotinoide können für sich allein oder auch in Form von Mischungen verwendet werden, je nach Anwendungszweck, bzw. je nach der gewünschten Färbung. Der Ausdruck "hochsiedendes Oel" bedeutet im Rahmen der vorliegenden Erfindung insbesondere Triglyceride von Fettsäuren mit etwa 8-22 Kohlenstoffatomen, vorzugsweise mit etwa 16-20 Kohlenstoffatomen wie etwa Speiseöle, z.B. Arachisöl, Maisöl, Sonnenblumenöl und gehärtetes Kokosnussöl. Weiterhin sind hierunter Triglyceridgemische mit 8-12 Kohlenstoffatomen zu verstehen, insbesondere Triglyceride gesättigter Fettsäuren mit 8-12 Kohlenstoffatomen wie z.B. die unter den Markennamen MIGLYOL 812 oder MYRITOL 318 erhältlichen Produkte.

Derartige Oele haben in der Regel einen Siedepunkt von wenigstens etwa 200° C und dienen in dem erfindungsgemässen Verfahren als "Lösungsmittel/Trägermaterial" für die eingesetzten Carotinoide.

Das Suspendieren eines Carotinoides in einem hochsiedenden Oel kann in an sich bekannter Weise erfolgen, beispielsweise durch Vermischen in einem Rührkessel. Sofern die erhaltene Suspension zu grobteilig ist, kann diese durch Mahlen z.B. in einer Kugelmühle in eine Suspension übergeführt werden, in welcher ca. 90 % der Partikel eine Grösse von weniger als 15 µm aufweisen. Die Herstellung dieser Suspension erfolgt zweckmässig unter Inertgas, wie etwa Argon oder Stickstoff. Da die Carotinoide, wie eingangs erwähnt, oxydationsempfindlich sind, wird dieser Suspension zweckmässig ein Antioxydationsmittel zugesetzt. Als solche kommen hier insbesondere in Frage Tocopherole, insbesondere dl-$\alpha$-Tocopherol, BHT (tert.-Butylhydroxytoluol) oder auch BHA (tert.-Butylhydroxyanisol) und dergleichen. Die Konzentration der erwähnten Suspension ist abhängig vom jeweils verwendeten Carotinoid, sowie auch von dem vorgesehenen Verwendungszweck des Endproduktes. In der Regel liegen diese Konzentrationen zwischen 10-50 Gew. %.

Das in Kontakt bringen der Suspension mit dem überhitzten Wasserdampf kann in einem geeigneten Mixer, z.B. in einem Inline-Mixer erfolgen. Die Suspension wird dabei kontinuierlich in den Mixer dosiert und gleichzeitig wird überhitzter Wasserdampf eingespeist. Die Temperatur des Dampfes beträgt hierbei zweckmässig von 180° C bis 230° C, vorzugsweise von 190° bis 210° C, und der Druck liegt zwischen 10 bar und 30 bar, vorzugsweise bei 12 bar bis 19 bar. Im Falle von z.B. $\beta$-Carotin hat der Dampf beim Eintritt in den Mixer vorzugsweise eine Temperatur von 200° - 205° C, bei einem Druck von 16 bar, gemessen am Dampfeinlass. Die Dampfmenge wird hier auch so geregelt, dass die Temperatur des Gemisches am Ausgang des Mixers 180°-190° C beträgt. Um zu verhindern, dass es bei den erwähnten hohen Temperaturen zu grossen Verlusten an Carotinoiden kommt, sowie auch um allfällige Isomerisierungen - z.B. trans- zu cis-$\beta$-Carotin - möglichst steuern zu können, beträgt die Kontaktzeit von überhitztem Wasserdampf mit der Carotinoidsuspension in dem Mixer höchstens 30 Sekunden. Vorzugsweise liegt diese Kontaktzeit jedoch bei höchstens 15-20 Sekunden, bzw. bei höchstens 5-10, bzw. 1-2 Sekunden, und insbesondere unterhalb 0,5 Sekunden. Dies lässt sich insbesondere durch die Grösse des Mixers und durch Variation der Durchsatzmenge (Carotinoidsuspension + Wasserdampf) regulieren. Zudem muss zwecks rascher Temperaturerniedrigung das entstandene Oel-Wasser-Carotinoidgemisch sofort in eine wässrige Matrix eines Schutzkolloides emulgiert werden. Als Matrix wird hier zweckmässig eine wässrige Lösung eines Kolloides und eines Emulgators verwendet. Als Kolloide kommen insbesondere in Frage sämtliche, normalerweise als Schutzkolloide verwendbare Substanzen, wie etwa Gelatine, Gummi-arabicum, Milch- und Pflanzenproteine, Kohlenhydrate wie Zucker, Stärke und Stärkederivate, sowie auch Gemische hiervon. Vorzugsweise wird jedoch ein Gemisch von Gelatine und Zucker verwendet. Als Emulgatoren kommen in Frage die üblichen, für pharmazeutische Präparte bzw. für Lebensmittel zugelassenen Produkte, wie etwa Sorbitanderivate, Glycerinmonosterat, Citronensäureester und insbesonders Ascorbylpalmitat.

Das Emulgieren kann in an sich bekannter Weise erfolgen, wie etwa durch Rühren oder mittels Ultraschall. In der so erhaltenen Emulsion beträgt die mittlere Teilchengrösse der inneren Phase (Oel in Wasser) 0,5 - 1 µm. Diese Emulsion wird anschliessend auf 50° - 70° C abgekühlt und in einer zweiten Homogenisierstufe feindispergiert, d.h. auf Teilchengrössen der inneren Phase von 0,1 - 0,3 µm. Dieses Homogenisieren kann wiederum in an sich bekannter Weise erfolgen. Im Anschluss an diese zweite Homogenisierstufe wird die Emulsion dann in an sich bekannter Weise versprüht und in Trockenpräparate übergeführt.

Das gesamte erfindungsgemässe Verfahren kann sowohl kontinuierlich als auch batchweise durchgeführt werden.

Das erfindungsgemässe Verfahren kann beispielsweise in einer Apparatur gemäss Schema I wie folgt durchgeführt werden:

In einem Rührkessel (1) wird eine Suspension eines Carotinoides in einem hochsiedenden Oel hergestellt, gegebenenfalls unter Beigabe eines Antioxydans. Die Suspension wird dann in einer Kugelmühle (2) gemahlen, bis etwa 90% der Teilchen eine Grösse von weniger als 15 µm aufweisen. Anschliessend wird diese Suspension mittels einer Pumpe (4) in den Mixer (5) dosiert. Gleichzeitig wird in den Mixer überhitzter Wasserdampf über den Dampfeinlass (6) eingespeist. Die Dampfmenge wird hierbei so reguliert, dass am Austritt des Mixers die gewünschte Temperatur herrscht. Die Verweilzeit der Carotinoidsuspension in dem Mixer beträgt höchstens 30 Sekunden. Parallel hierzu wird in einem heizbaren Kessel (3) eine wässrige Matrix aus einem Schutzkolloid und einem Emulgator hergestellt und über eine Pumpe (13) in den Homogenisierkessel (7) gepumpt. Hierin wird sodann die Matrix mit dem Oel-Wasser-Carotinoidgemisch aus dem Mixer (5) emulgiert, abgekühlt und homogenisiert. Die erhaltene Emulsion wird anschliessend mittels eines Ventils (9) auf Atmosphärendruck entspannt, dann in einem Wärmetauscher (10) abgekühlt und in einem Homogenisator (11) feindispergiert. Anschliessend wird die erhaltene Dispersion in einem Sprühturm (12) versprüht und getrocknet.

Beispiel 1

1) In einem Rührkessel (1) wurden 48 kg β-Carotin krist. in einer Mischung aus 6.2 kg dl-α-Tocopherol und 54.9 kg MIGLYOL 812 (Triglycerid gesättigter Fettsäuren mittlerer Kettenlänge, insbesondere $C_8$ und $C_{10}$) suspendiert. Diese Suspension wurde in einer Kugelmühle (2) so gemahlen, dass 90% der Teilchen <15μm waren.

2) In einem zweiten Rührkessel (3) wurden 30.8 kg Zucker und 109.5 kg Gelatine in 213.8 kg Wasser gelöst. Der pH Wert wurde mit Natronlauge auf 7.0 eingestellt. Nun wurden 5.5 kg Ascorbylpalmitat eingetragen und der pH Wert wurde dabei ständig auf 7.0 eingestellt, bis sich das Ascorbylpalmitat gelöst hatte.

3) In den Inlinemixer (5) wurden mittels einer Zahnradpumpe (4) 20-22 kg/h der gemäss 1) hergestellten β-Carotinsuspension dosiert. Gleichzeitig wurde der Mixer mit Dampf (Menge ca. 6 kg/h) von 16 bar (6) und einer Temperatur von 200°C gespeist. Die Dampfmenge wurde so geregelt, dass die Temperatur am Ausgang des Inlinemixers 185-186°C betrug. Der Druck des Dampfes vor dem Regelventil betrug 30 bar. Die Verweilzeit der β-Carotinsuspension im Inlinemixer bei einer Temperatur von 185-186°C betrug <0.5 Sekunden. Sofort anschliessend wurde die heisse, homogene Mischung von β-Carotin, MIGLYOL 812, Tocopherol und Wasser mit der ca. 48°C warmen , gemäss 2) hergestellten Matrixlösung in dem Homogenisierkessel (7) gemischt. Die Matrixlösung wurde hierbei über eine Pumpe (13) in einer Menge von 186 kg/h in das Homogenisiergefäss (7) zudosiert. Dabei erniedrigte sich die Temperatur der β-Carotin/MIGLYOL/Wasser-Mischung von 185-186°C schlagartig auf 74-76°C, wobei sich zunächst eine grobteilige Emulsion bildete, die durch Behandlung in dem Homogenisator (8) verfeinert wurde. Die mittlere Verweilzeit im Homogenisierkessel (7) betrug 0,3 Minuten. Die Emulsion wurde dann über ein Druckhalteventil (9) und einen Rohrkühler (10) kontinuierlich ausgetragen und dabei auf ca. 55-60°C gekühlt. Der Druck im Homogenisierkessel (7) betrug 15-16 bar.

Die so erhaltene β-Carotinemulsion wurde anschliessend im Homogenisator (11) zu einer Teilchengrösse der inneren Phase von 0.25 μm nachhomogenisiert. Anschliessend wurde die viskose Flüssigkeit durch Sprühen in ein Stärkebett (12) und anschliessendes Trocknen in ein stabiles Trockenpulver überführt. Der β-Carotingehalt des Trockenpulvers beträgt 7.0%, wobei 70% als trans-β-Carotin vorliegen.

Beispiel 2

In zu Beispiel 1 analoger Weise wurden β-Carotinpulver unter Einsatz der Oele Maisöl, Arachisöl bzw. gehärtetes Kokosnussöl hergestellt:

| | Versuch 1) | Versuch 2) | Versuch 3) |
|---|---|---|---|
| **ß-Carotinsuspension** | | | |
| – ß-Carotin krist. | 22.0 kg | 22.0 kg | 41.0 kg |
| – dl-α-Tocopherol | 2.9 kg | 2.9 kg | 4.7 kg |
| – Arachisöl | – | 75.1 kg | – |
| – Maisöl | 75.1 kg | – | – |
| – Kokosnussöl, gehärtet | | | 54.3 kg |
| **Matrix** | | | |
| – Wasser | 160.0 kg | 120.0 kg | 163.6 kg |
| – Zucker | 16.4 kg | 16.4 kg | – |
| – Gelatine | 58.4 kg | 58.4 kg | 90.0 kg |
| – Ascorbylpalmitat | 5.85 kg | 1.3 kg | 3.0 kg |
| – Sorbitlösung 70 %-ig | – | – | 43.4 kg |
| **Feedmengen Inlinemischer** | | | |
| – ß-Carotinsuspension | 22.0 kg/h | 19.5 kg/h | 21.0 kg/h |
| – Dampf | 6.0 kg/h | 6.0 kg/h | 6.0 kg/h |
| – Temperatur am Ausgang des Inlinemischers | 185-186°C | 186°C | 185-186°C |
| – Verweilzeit im Inlinemischer | <0.5 Sek. | <0.5 Sek. | <0.5 Sek. |
| **Homogenisierkessel** | | | |
| – Matrix | 70.0 kg/h | 56.0 kg/h | 143.8 kg/h |
| – Temperatur im Homogenisiergefäss | 86-88°C | 88°C | 82-84°C |
| – Verweilzeit im Homogenisierkessel | 0,6 Min. | 0,75 Min. | 0,7 Min. |
| **ß-Carotinpulver** | | | |
| – Gehalt an ß-Carotin | 6.7 % | 7.5 % | 7.5 % |
| – trans-ß-Carotin | 70.0 % | 68.0 % | 69.2 % |

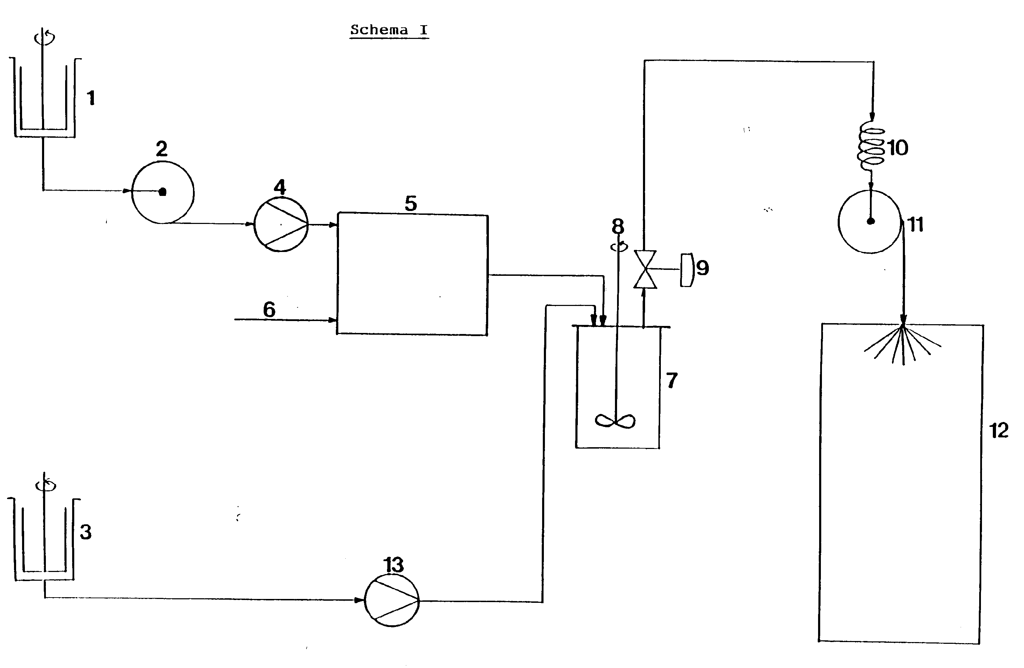

**Patentansprüche**

1. Verfahren zur Herstellung von kolloid-dispersen Carotinoidpräparaten, dadurch gekennzeichnet, dass man eine Suspension eines Carotinoides in einem hochsiedenden Oel während einer Zeitdauer von höchstens 30 Sekunden mit überhitztem Wasserdampf in Kontakt bringt, dass man das erhaltene Gemisch in eine wässrige Lösung eines Kolloides emulgiert und diese Emulsion anschliessend versprüht und trocknet.

2. Verfahren gemäss Anspruch 1, dadurch gekennzeichnet, dass man als hochsiedendes Oel ein Triglycerid von gesättigten Fettsäuren, Arachisöl, Maisöl oder gehärtetes Kokosnussöl verwendet.

3. Verfahren gemäss Anspruch 1 oder 2, dadurch gekennzeichnet, dass die Kontaktzeit zwischen Carotinoidsuspension und überhitztem Wasserdampf höchstens 15-20, bzw. 5-10, insbesondere 1-2 Sekunden und vorzugsweise weniger als 0,5 Sekunden beträgt.

4. Verfahren gemäss einem der Ansprüche 1-3, dadurch gekennzeichnet, dass man als Carotinoid β-Carotin verwendet.

## Claims

1. A process for the manufacture of colloid-dispersed carotenoid preparations, characterized by bringing a suspension of a carotenoid in a high-boiling oil into contact with superheated steam during a maximum period of 30 seconds, emulsifying the mixture obtained in an aqueous solution of a colloid and subsequently spraying and drying this emulsion.

2. A process according to claim 1, characterized in that a triglyceride of saturated fatty acids, groundnut oil, corn oil or hardened coconut oil is used as the high-boiling oil.

3. A process according to claim 1 or 2, characterized in that the contact time between the carotenoid suspension and superheated steam is a maximum of 15-20 or 5-10, especially 1-2, seconds and preferably less than 0.5 second.

4. A process according to any one of claims 1-3, characterized in that β-carotene is used as the carotenoid.

## Revendications

1. Procédé pour la préparation de compositions de caroténoïdes en dispersion colloïdale, caractérisé en ce que l'on met une suspension d'un caroténoïde dans une huile à haut point d'ébullition en contact avec de la vapeur d'eau surchauffée pendant une durée de 30 secondes au maximum, on émulsionne le mélange ainsi obtenu dans une solution aqueuse d'un colloïde et on pulvérise et sèche ensuite cette émulsion.

2. Procédé selon la revendication 1, caractérisé en ce que l'on utilise en tant qu'huile à haut point d'ébullition un triglycéride d'acides gras saturés, de l'huile d'arachide, de l'huile de maïs ou de l'huile de coco durcie.

3. Procédé selon la revendication 1 ou 2, caractérisé en ce que la durée de contact entre la suspension de caroténoïde et la vapeur d'eau surchauffée est au maximum de 15 à 20, ou de 5 à 10, plus spécialement de 1 à 2 secondes et de préférence de moins de 0,5 seconde.

4. Procédé selon l'une des revendications 1 à 3, caractérisé en ce que le caroténoïde est le bêta-carotène.

Schema I

EP 0 410 236 B1